(19) Europäisches Patentamt
European Patent Office
Office européen des brevets

(11) Veröffentlichungsnummer: **0 258 800**
**A2**

(12) # EUROPÄISCHE PATENTANMELDUNG

(21) Anmeldenummer: **87112366.7**

(22) Anmeldetag: **26.08.87**

(51) Int. Cl.[4]: **C07D 451/04**, A61K 31/46

(30) Priorität: **30.08.86 DE 3629598**

(43) Veröffentlichungstag der Anmeldung:
**09.03.88 Patentblatt 88/10**

(84) Benannte Vertragsstaaten:
**AT BE CH DE ES FR GB GR IT LI LU NL SE**

(71) Anmelder: **BOEHRINGER INGELHEIM KG**

**D-6507 Ingelheim am Rhein(DE)**

(84) **BE CH DE ES FR GR IT LI LU NL SE AT**

(71) Anmelder: **Boehringer Ingelheim International G.m.b.H**

D-6507 Ingelheim am Rhein(DE)

(84) **GB**

(72) Erfinder: **Sobotta, Rainer, Dr.**
**Ludwig Richter Strasse 6**
**D-6507 Ingelheim am Rhein(DE)**
Erfinder: **Langbein, Adolf, Dr.**
**Am Goldberg 6**
**D-6535 Gau-Algeheim(DE)**
Erfinder: **Merz, Herbert, Dr.**
**Rotweinstrasse 53**
**D-6507 Ingelheim am Rhein(DE)**
Erfinder: **Bauer, Rudolf, Dr.**
**Aarstrasse 4**
**D-6200 Wiesbaden(DE)**
Erfinder: **Mierau, Joachim, Dr.**
**An den Weiden 3**
**D-6500 Mainz 33(DE)**

(54) **3-Brom-2,6-dimethoxibenzamide, ihre Herstellung und Verwendung.**

(57) Die neuen Verbindungen der Formel

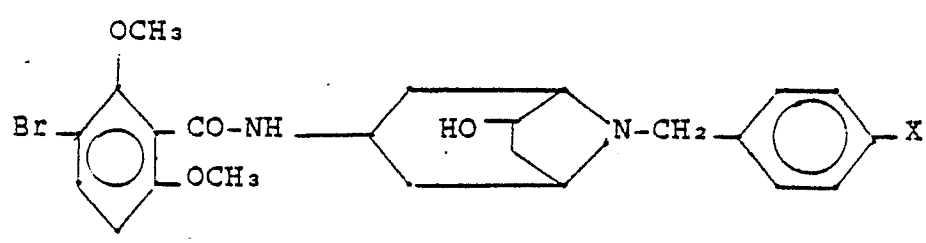

(I)

(X gleich Wasserstoff, Fluor, Chlor) können nach üblichen Verfahren hergestellt und als Arzneistoffe verwendet werden.

EP 0 258 800 A2

## 3-Brom-2,6-dimethoxibenzamide, ihre Herstellung und Verwendung

Die Erfindung betrifft neue 3-Brom-2,6-dimethoxibenzamide, ihre Herstellung nach üblichen Methoden und ihre Verwendung als Arzneistoffe.

Die neuen Verbindungen entsprechen der Formel

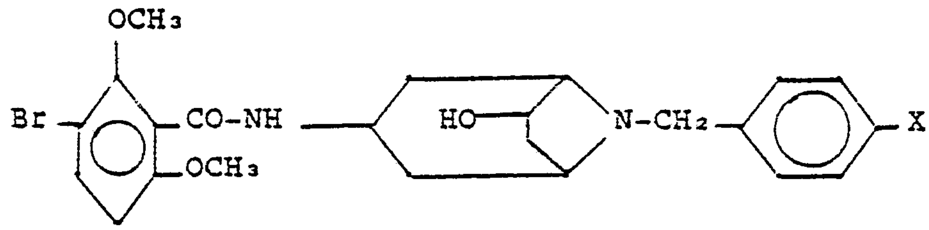

(I)

worin X für Wasserstoff, Fluor oder Chlor steht; sie können als freie Basen oder als Säureadditionssalze vorliegen. Die Formel I umfaßt sowohl die Enantiomerengemische als auch die reinen Enantiomeren.

Zur Herstellung kann ein entsprechendes 3-Amino-6-hydroxy-nortropan der Formel

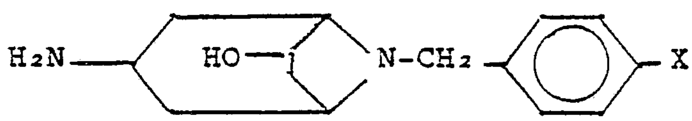

(II)

worin X die obige Bedeutung hat, mit einem Benzoesäurederivat der Formel

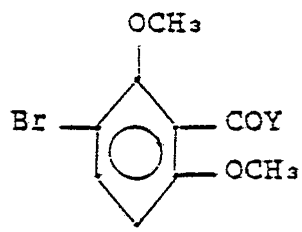

(III)

worin Y für eine mit einem Wasserstoffatom der Aminogruppe von II als HY abspaltbare Gruppe steht, umgesetzt werden.

Wird bei der Aufarbeitung das Reaktionsprodukt I als freie Basen erhalten, kann es anschließend in üblicher Weise in ein gewünschtes Säureadditionssalz, vorzugsweise mit einer physiologisch unbedenklichen Säure, überführt werden. Erhaltene Enantiomerengemische können nach üblichen Methoden aufgetrennt werden. Auch können Verbindungen der Formel II in Form der reinen Enantiomeren eingesetzt werden. Beispiele für die Gruppe Y sind OH, Chlor, Brom, $CNCH_2O$-, Ethoxicarbonyloxi, Imidazolyl.

Bei Verwendung von Dimethoxibenzoyl-halogeniden der Formel III verwendet man die berechnete Menge des Acylierungsmittels oder einen Überschuß davon und arbeitet zweckmäßig in Gegenwart eines säurebindenden Stoffes wie z.B. Dicyclohexylethylamin, Natriumcarbonat, Kaliumcarbonat, Calciumoxid oder vorzugsweise Triethylamin. Obwohl auf Lösungsmittel verzichtet werden kann, ist die Durchführung in inerten Lösungsmitteln wie Chloroform, Toluol, Nitromethan, Tetrahydrofuran, Dimethylformamid oder vorzugsweise Methylenchlorid vorteilhaft. Die Reaktionstemperatur ist in weiten Grenzen variabel. Zweckmäßig sind Temperaturen um 20°C oder darunter.

Die Acylierung der Verbindungen der Formel II mittels eines Carbonsäure-imidazolids erfolgt in der Weise, daß die entsprechende 2,6-Dimethoxibenzoesäure zunächst mit Carbonyldiimidazol und daran anschließend mit dem Amin umgesetzt wird. Man verwendet die berechnete Menge der Carbonsäure und äquivalenten Mengen Carbonyldiimidazol. Die Reaktion erfolgt in inerten Lösungsmitteln. Als besonders günstig hat sich Tetrahydrufuran erwiesen. Die Reaktionstemperatur ist in Grenzen variabel. Die Reaktion wird am besten bei O bis 10°C durchgeführt.

Die nach dem Verfahren erhaltenen Reaktionsprodukte werden aus den Reaktionsansätzen nach bekannten Methoden isoliert. Gewünschtenfalls können die erhaltenen Rohprodukte noch unter Verwendung besonderer Verfahren, z.B. durch Säulenchromatographie, gereinigt werden, ehe man sie in Form der Basen oder geeigneter Säureadditionsverbindungen kristallisiert.

Die erfindungsgemäßen 2,6-Dimethoxibenzamide der Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure Zimtsäure, Salicylsäure, Ascorbinsäure, Methansulfonsäure, 8-Chlortheophyllin.

Die Ausgangsstoffe der Formeln II und III sind bekannt oder sonst nach üblichen Verfahren erhältlich.

Ausgangsverbindungen der Formel II können nach folgendem Syntheseschema erhalten werden (für X = F, Cl):

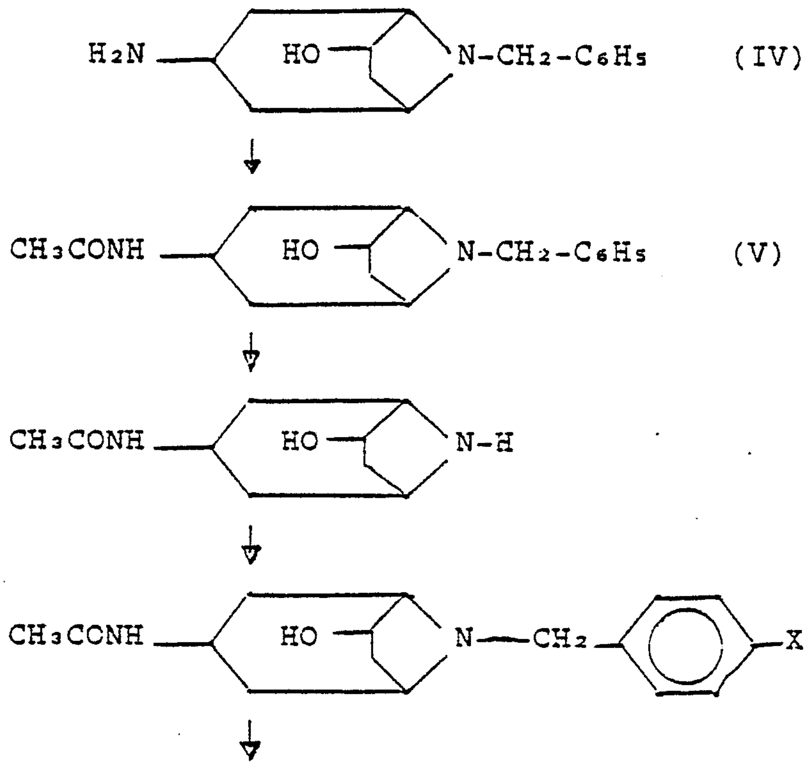

(IV)

(V)

(VI)

(VII)

II

Die Verbindung IV ist aus der EP A 83 302 493.8 bekannt.

Die neuen Verbindungen der Formel I zeigen das typische Wirkungsbild der Neuroleptika und können daher als ZNS-dämpfende Mittel eingesetzt werden. Gegenüber bekannten strukturähnlichen Verbindungen ist die besonders günstige Differenzierung zwischen antidopaminerger Haupt-und dyskinetischen Nebenwirkungen hervorzuheben.

Pharmakologisch wurden die neuen Verbindungen in zahlreichen Testen untersucht.

Im Apomorphin-Klettertest an der Maus (B. Costall et al., Europ. J. Pharmacol. 50, 39 ff (1978), modifiziert) zeigen sie starke apomorphinantagonistische wirkungen, die die der oben genannten Vergleichsverbindungen teilweise beträchtlich übertreffen. Diese Eigenschaften lassen nach dem Stand der Technik neuroleptische Wirkungen am Menschen erwarten. Die Eigenschaften waren aufgrund der engen Strukturverwandtschaft mit den aus der EU-PS Nr. 4 831 bekannten Verbindungen überraschend und nicht vorhersehbar. Aussagen über Nebenwirkungen wie Sedation oder Störung der Motokoordination konnten mit Hilfe folgender Testanordnungen gemacht werden: Die Motilitätsmessung an Mäusen nach T.H.

SVENSSON und G. THIEME, Psychopharmacol. (Berlin) 14, 157 ff (1969), modifiziert, gibt Hinweise auf Sedation. Der Ataxietest am rotierenden Stab (Rotarod) bei Mäusen nach N.W. DUNHAM und T.S. MIYA, J. Amer. Pharm. Accos. Sci. Ed. 46, 208 ff (1957), modifiziert, erlaubt Aussagen über Störungen der Motokoordination.

Die dyskinetischen Effekte gehören zu den störendsten Nebenwirkungen der Neuroleptika. Dazu zählen parkinsonähnliche Erscheinungen wie Tremor, Rigor und Akinese, aber auch Dyskinesien im Mund-Zungen-Schlund-Bereich.

Die Untersuchung derartiger Symptome im Tiermodell ist an mit Haloperidol sensibilisierten Rhesus-Affen möglich. Derartige Tiere zeigen nach Gabe von Neuroleptika, die am Menschen extrapyramidal motorische Wirkungen besitzen, typische Dyskinesien im Mund-und Zungenbereich (nach J. Liebmann und R. Neale, Psychopharmacology 68, 25-29 (1980), modifiziert).

Bezüglich der Nebenwirkungen verhalten sich die zu schützenden Verbindungen günstiger als die Vergleichssubstanzen.

Die Überlegenheit der erfindungsgemäßen Verbindungen gegenüber der strukturell ähnlichsten Verbindung aus dem Stand der Technik, nämlich N-(8-Benzyl-3-nortropanyl)-2,6-dimethoxi-3-brombenzamid (Verbindung A; vgl. DE-A 3 340 629, Beispiel 2), zeigt sich in den pharmakologischen Daten der nachstehenden Tabelle:

| Verbindung | Klettertest $ED_{50}$ [mg/kg] p.o. nach 1 h | Dyskinesie Schwellendosis p.o. [mg/kg] | Dyskinesie-Index I : II | $LD_{50}$ (Maus) p.o. [mg/kg] |
|---|---|---|---|---|
| A (bekannt) | 1,8 | 10 | 0,18 | ~300 |
| erfindungsgemäß: Formel I, | | | | |
| X = H | 1,2 | > 15 | < 0,08 | ~300 |
| X = F | 0,63 | 8 | 0,078 | >300 <600 |
| X = Cl | 0,85 | 30 | 0,028 | > 1000 |

Die erfindungsgemäßen Verbindungen können allein oder in Kombination mit anderen Wirkstoffen zur Anwendung gelangen. Geeignete Anwendungsformen sind beispielsweise Tabletten, Kapseln, Zäpfchen, Lösungen, Säftr, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln wie Maisstärke, Alginsäure, Bindemitteln wie Stärke oder Gelatine, Schmiermitteln wie Magnesiumstearat oder Talkum und/oder Mitteln zur Erzielung eines Depoteffektes wie Carboxipolymethylen, Carboximethylcellulose, Celluloseacetatphthalat oder Polyvinylacetat erhalten werden.

Die neuen Verbindungen können enteral oder parenteral angewandt werden. Bei oraler Verabreichung können Einzeldosierungen von 0,5 - 10 mg, vorzugsweise 1 - 5 mg in Betracht.

Pharmazeutische Formulierungsbeispiele

a) Dragées

1 Dragéekern enthält:

| | |
|---|---|
| Wirkstoff gemäß Formel I | 2,0 mg |
| Milchzucker | 28,5 mg |
| Maisstärke | 17,0 mg |
| Gelatine | 2,0 mg |
| Magnesiumstearat | 0,5 mg |
| | 50,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxid, Talkum und Gummi arabicum aufgebracht wird. Die fertigen Dragées werden mit Bienenwachs poliert. Dragée-Endgewicht: 100 mg

b) Tabletten

| | |
|---|---|
| Wirkstoff gemäß Formel I | 2,0 mg |
| Milchzucker | 55,0 mg |
| Maisstärke | 38,0 mg |
| lösliche Stärke | 4,0 mg |
| Magnesiumstearat | 1,0 mg |
| | 100,0 mg |

Herstellung

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die 2 mg Wirkstoff enthalten.

c) Suppositorien

1 Zäpfchen enthalt:

Wirkstoff gemäß Formel I 1.0 mg

Zäpfchenmasse 1699.0 mg

Herstellung:

Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) Ampullen

Wirkstoff gemäß Formel I 2,0 mg
Natriumchlorid 18,0 mg
destilliertes Wasser ad 2,0 ml

Herstellung:

Wirkstoff und Natriumchlorid werden in Wasser gelöst, die Lösung frei von suspendierten Partikeln filtriert und in 2 ccm-Ampullen unter aseptischen Bedingungen abgefüllt. Zuletzt werden die Ampullen sterilisiert und verschlossen. Jede Ampulle enthält 2 mg Wirkstoff.

Die nachstehenden Beispiele sollen die Erfindung näher erläutern.

Herstellung von Ausgangsstoffen

a) 3-Acetamido-6-hydroxy-8-benzylnortropan

Zu einer Lösung von 30 g 3-Amino-6-hydroxy-8-benzylnortropan (0.129 mol) und 13.1 g Triäthylamin (0.129 mol) in 250 ml Methylenchlorid werden 10,1 g Acetylchlorid (0,129 mol) bei 20° zugetropft. Es wird anschließend 3 Stunden am Rückfluß gekocht. Nach dem Abkühlen wird die Lösung einmal mit 200 ml Natronlauge (10%ig) und dreimal mit je 200 ml Wasser gewaschen. Nach Trocknung der Methylenchlorid-Phase über Natriumsulfat erhält man durch Abdestillieren des Lösungsmittels 28,4 g weiße Kristalle 80,2% vom Schmelzpunkt 108°C.

b) 3-Acetamido-6-hydroxynortropan

28,4 g 3-Acetamido-6-hydroxy-8-benzylnortropan (0,104 mol), in 300 ml Methanol gelöst, werden in einem Schüttelautoclav nach Zugabe von 2,8g Palladium auf Kohle (5%ig) bei 20°C und einem Wasserstoff-Druck von 5 bar hydriert. Nach Beendigung der Wasserstoffaufnahme wird die Lösung vom Katalysator durch Filtration abgetrennt und im Vakuum eingeengt. Der zunächst ölige Rückstand wird in einem Gemisch aus Essigester und Äthanol (9 : 1) kristallisiert. Es werden 16,2 g weiße Kristalle 85% d. Th. mit einem Schmelzpunkt von 173°C erhalten.

c) Acetamido-6-hydroxy-8-(4-chlorbenzyl)-nortropan

Zu einer Lösung von 5,53 g 3-Acetamido-6-hydroxynortropan (0.03 mol) in 50 ml Lösungsmittelgemisch (Dimethylformamid/Tetrahydiofuran; 4 : 1) werden 5,04 g Natriumbicarbonat (0,06 mol) zugegeben. Nach dem Zutropfen von 5.31 g 4-Chlorbenzylchlorid (0,033 mol) gelöst in 10 ml Lösungsmittelgemisch wird 7 Stunden unter Rückfluß gekocht. Nach dem Abkühlen wird das Lösungsmittelgemisch in Vakuum abdestilliert. Der Rückstand wird mit 200 ml Wasser versetzt und dreimal mit je 100 ml Methylenchlorid extrahiert. Die vereinigten Methylenchlorid-Phasen werden über Natriumsulfat getrocknet und im Vakuum eingeengt. Man erhält 8,9 g weiße Kristalle 96% d. Th. mit einem Schmelzpunkt von 105°C.

d) Acetamido-6-hydroxy-8-(4-fluorbenzyl)-nortropan

Die Durchführung und Aufarbeitung erfolgt wie bei Herstellung von 3-Acetamido-6-hydroxy-8-(4-chlorbenzyl)-nortropan . Aus 5,53 g 3-Acetamido-6-hydroxy-nortropan (0,03 mol) und 6,08 g 4-Flourbenzylbromid (0.033 mol) erhält man 5,7 g weiße Kristalle (65 % d. Th.) mit dem Schmelzpunkt 125°C.

e) 3-Amino-6-hydroxy-8-(4-fluorbenzyl)-nortropan

8,9 g 3-Acetamido-6-hydroxy-8-(4-flourbenzyl)-nortropan wird in 100 ml 6N Salzsäure 7 Stunden unter Rückfluß zum Sieden erhitzt. Nach dem Abkühlen wird im Vakuum zur Trockene eingeengt. Der Rückstand wird in Alkohol gelöst und durch Zugabe von Essigester bis zum Verhältnis 1 : 1 zur Kristallisation gebracht. Es werden 4,74 g weiße Kristalle des Dihydrochlorids mit einem Schmelzpunkt von 250°C erhalten: Ausbeute: 77.7% der Theorie.

Zu 4,74 g Dihydrochlorid (14,8 mmol), in Methanol gelöst, werden 1,6 g Natriummethanolat (29,6 mmol) gegeben und 10 min gerührt. Die entstandene Suspension wird über Kieselgur filtriert und das Filtrat im Vakuum zur Trockene eingeengt. Es werden 3,7 g weiße Kristalle der Base mit dem Schmelzpunkt 130°C erhalten.

f) 3-Amino-6-hydroxy-8-(4-chlorbenzyl)-nortropan

Die Durchführung und Aufarbeitung erfolgt wie bei der Herstellung von 3 Amino-6-hydroxy-8-(4-fluorbenzyl)-nortropan

Aus 8,9 g 3-Acetamido-6-hydroxy-8-(4-chlorbenzyl)-nortropan werden 5,38 g weiße Kristalle 47,7 % d.Th. des Dihydrochlorids mit dem Schmelzpunkt 300°C erhalten. Die Freisetzung der Base ergibt 4,6 g beige Kristalle mit dem Schmelzpunkt 145°C

Herstellung der Endprodukte

Beispiel 1

N-(8-Benzyl-6-hydroxy-3-nortropanyl)-2,6-dimethoxy-3-brombenzamid-fumarat

Zu 5,22 g 2,6-Dimethoxy-5-brombenzoesäure (20 mmol), in 60 ml Methylenchlorid gelöst, wird ein Tropfen Dimethylformamid zugegeben und langsam 3,5 ml Oxalchlorid bei 20°C zugetropft. Wenn das Reaktionsgemisch nur noch wenig Gas entwickelt, wird noch 20 Minuten zum Sieden erhitzt. Das Lösungsmittel wird abdestilliert. Den Rückstand löst man in 40 ml trockenem Methylenchlorid und tropft ihn bei Raumtemperatur zu einer Lösung aus 4,64 g 3-Amino-6-hydroxy-8-benzyl-nortropan (20 mmol) und 7,2 ml Triethylamin in 60 ml Methylenchlorid. Man läßt 3 Stunden nachreagieren und arbeitet dann auf. Die Lösung wird mit 100 ml Methylenchlorid verdünnt, dann zweimal mit je 100 ml Wasser und anschließend mit Natronlauge (10%ig) gewaschen.

Die organische Phase wird über Natriumsulfat getrocknet und eingeengt. Der Rückstand wird in Methanol gelöst und durch Zugabe von 2,32 g Fumarsäure (m Methanol) zur Kristallisation des Salzes gebracht. Man erhält 8,6 g weiße Kristalle (72,7 % d. Th.) Schmelzpunkt 144°C. Elementaranalyse:

| | | | | |
|---|---|---|---|---|
| $C_{23}H_{27}BrN_2O_4$ | ber. | C 54.83 | H 5,28 | N 4,74 |
| 591,47 | gef. | C 54,92 | H 5,38 | N 4,70 |

Beispiel 2

N-[8-(4-Fluorbenzyl)-6-hydroxy-6-nortropanyl]-2,6-dimethoxy -3-brombenzamid-hydrochlorid

Durchführung und Aufarbeitung wie beim Beispiel 1 Aus 1,93 g 2,6-Dimethoxy-5-brombenzoesäure, 0,94 g Oxalchlorid, 0,75 g Triäthylamin und 1,85 g 3-Amino-6-hydroxy-8-(4-fluorbenzyl)-nortropan erhält man die Rohbase, die mit methanolischer Salzsäure 1,8 g weiße Kristalle mit dem Schmelzpunkt 140°C in einer Ausbeute von 44% der Theorie ergibt.

$C_{23}H_{26}BrFN_2O_4$ x HCL

| | | | | |
|---|---|---|---|---|
| | ber. | C 52.14 | H 5,14 | N 5,29 |
| 529,84 | gef. | C 51,53 | H 5,53 | N 4,94 |

## Beispiel 3

N-[8-(4-Chlorbenzyl)-6-hydroxy-3-nortropanyl]-2,6-dimethoxy-3-brombenzamid-hydrochlorid

Durchführung und Aufarbeitung wie beim Beipsiel 1. Aus 11,55 g 2,6-Dimethoxy-5-brombenzoesäure. 5,63 g Oxalychlorid, 4,49 g 3-Amino-6-hydroxy-8-(4-chlorbenzyl)-nortropan in 200 ml Methylenchlorid werden 17 g (75% d. Th.) weiße Kristalle der Base, Schmelzpunkt 179°C erhalten. Mit methanolischer Salzsäure bilden sich weiße Kristalle des Hydrochlorids mit dem Schmelzpunkt von 162-165°C.

Elementaranalyse

$C_{23}H_{26}BrClN_2O_4$ x HCl

| | | | | |
|---|---|---|---|---|
| | ber. | C 50.57 | H 4,98 | N 5,13 |
| (546.30) | gef. | C 49,96 | H 5,49 | N 4,74 |

(+)-N-[8-(4-Chlorbenzyl)-6-hydroxy-3-nortropanyl]-2,6-dimethoxy-3-brombenzamid-hydrochlorid

10 g der Base (19.58 mmol) und 2,97 g D (-) Mandelsäure (19,58 mmol) werden in ca. 15 ml Äthanol gelöst. Danach gibt man soviel Äther zu (ca. 45 ml) bis eine leichte Trübung entsteht. Über Nacht bilden sich bei 20°C erste Kristalle; nach weiterem Stehenlassen (72 Stunden) ist die Kristallisation abgeschlossen. Die klare überstehende Lösung wird abgedekantiert. Die Kristalle werden zweimal mit Äther-Äthanol-Gemisch (4 : 1) gewaschen, abgesaugt und getrocknet. Man erhält 6,5 g weiße Kristalle des Mandelats.

Das rohe Mandelat wird zur weiteren Reinigung zunächst mit Ammonialk (10%ig) in die Base überführt, diese mit Essigester extrahiert, getrocknet (Natriumsulfat) und im Vakuum eingeengt. Man erhält 4,7 g weiße Kristalle. Erneute Salzbildung mit D (-)-Mandelsäure und anschließende Rückführung in die Base liefern 2,06 g des reinen (+)-Enaniomers mit dem spezifischen Drehwert $[\alpha]^{25}$ von + 11,8° (c=0,01; Methanol).

Mit methanolischer Salzsäure bilden sich 2,1 g weiße Kristalle des (+)-Hydrochlorids, Schmelzpunkt 162 - 175°C.

## Ansprüche

1. Verbindungen der Formel

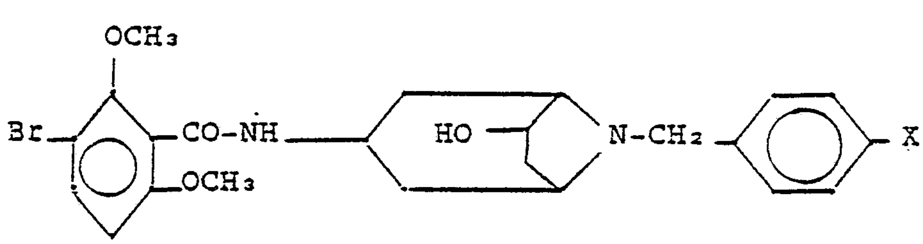

(I),

worin X für Wasserstoff, Fluor oder Chlor steht, in Form von Enantiomerengemischen oder reiner Enantiomerer, jeweils als freie Basen oder als Säureadditionssalze.

2. Arzneimittel, gekennzeichnet durch einen Gehalt an einer Verbindung nach Anspruch 1 neben üblichen Hilfs-und/oder Trägerstoffen.

3. Verwendung von Verbindungen nach Anspruch 1 als Neuroleptika.

4. Verfahren zur Herstellung von Verbindungen der Formel

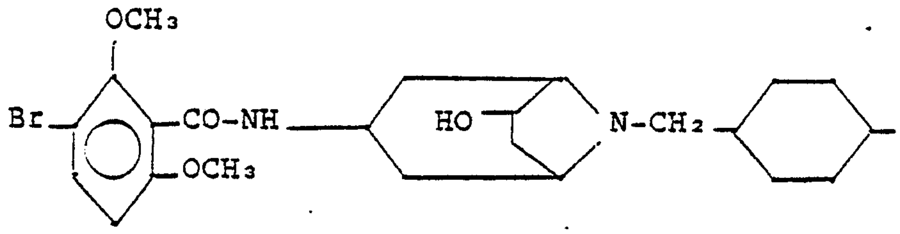

(I),

worin X für Wasserstoff, Fluor oder Chlor steht, in Form von Enantiomerengemischen oder reiner Enantiomerer, jeweils als freie Basen oder als Säureadditionssalze, nach üblichen Methoden, dadurch gekennzeichnet, daß man eine Verbindung der Formel

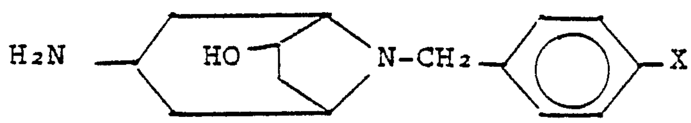

(II),

worin X Wasserstoff, Fluor oder Chlor bedeutet, mit einer Verbindung der Formel

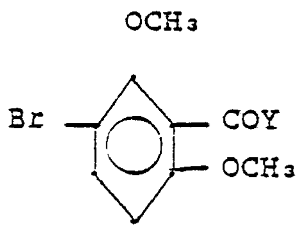

(III),

worin Y für eine mit einem Wasserstoffatom der Aminogruppe von II als HY abspaltbare Gruppe steht, umsetzt,
und daß man gewünschtenfalls ein gegebenenfalls erhaltenes Enantiomerengemisch in die Enantiomeren auftrennt und/oder zunächst erhaltene Basen in Säureadditionssalze, zunächst erhaltene Säureadditionssalze in freie Basen überführt.

5. Verfahren nach Anspruch 4, dadurch gekennzeichnet, daß man die Verbindung der Formel II in Form eines der Enantiomeren einsetzt.

6. N-(8-Benzyl-6-hydroxy-3-nortropanyl)-2,6-dimethoxy-3-brombenzamid, in Form der Enantiomerengemischen und reiner Enantiomerer, jeweils als freie Basen und Säureadditionssalze.

7. N-[8-(4-Fluorbenzyl)-6-hydroxy-3-nortropanyl]-2,6-dimethoxy-3-brombenzamid, in Form der Enantiomerengemischen und reiner Enantiomerer, jeweils als freie Basen und Säureadditionssalze.

8. N-[8-(4-Chlorbenzyl)-6-hydroxy-3-nortropanyl]-2,6-dimethoxy-3-brombenzamid, in Form der Enantiomerengemischen und reiner Enantiomerer, jeweils als freie Basen und Säureadditionssalze.